# EUROPEAN PATENT SPECIFICATION

(11) **EP 3 218 060 B1**
(45) Date of publication and mention of the grant of the patent: **30.10.2019**
(21) Application number: 15859189.1
(22) Date of filing: 04.11.2015
(51) Int. Cl.: A61K 9/12, A61K 9/08, A61P 29/00, A61K 31/19, A61K 47/10, A61K 47/12, A61K 47/14

(54) **SPRAYABLE ANALGESIC COMPOSITIONS**
SPRÜHBARE ANALGETISCHE ZUSAMMENSETZUNGEN
COMPOSITIONS ANALGÉSIQUES PULVÉRISABLES

(30) Priority: 10.11.2014 US 201462077581 P
(43) Date of publication of application: 20.09.2017
(73) Proprietor: Achelios Therapeutics, Inc., Durham, North Carolina 27713 (US)
(72) Inventor: BUYUKTIMKIN, Servet, San Diego, California 92130 (US); BUYUKTIMKIN, Nadir, San Diego, California 92130 (US); YEAGER, James L., Lake Forest, Illinois 60045 (US)
(74) Representative: Schnappauf, Georg
(86) International application number: PCT/US2015/058927
(87) International publication number: WO 2016/077111

(56) References cited:
- WO-A2-2007/070694
- US-A1- 2003 161 867
- US-A1- 2004 126 415
- US-A1- 2007 189 978
- US-A1- 2007 196 323
- US-A1- 2013 243 701
- SEBASTIANI P ET AL: "Effect of lactic acid and iontophoresis on drug permeation across rabbit ear skin", INTERNATIONAL JOURNAL OF PHARMACEUTICS, ELSEVIER, NL, vol. 292, no. 1-2, 23 March 2005 (2005-03-23), pages 119-126, XP027623889, ISSN: 0378-5173 [retrieved on 2005-03-23]

## Description

### FIELD OF INVENTION

This invention relates to analgesic compositions. More particularly, this invention relates to sprayable compositions containing nonsteroidal anti-inflammatory drugs.

### BACKGROUND OF THE INVENTION

Nonsteroidal anti-inflammatory drugs (NSAIDs) are known medications with analgesic and antipyretic effects. NSAIDs are used to treat pain and discomfort such as muscle strain/sprain, fever, inflammation such as rheumatoid arthritis, joint pain, and the like.

The document WO 2007/070694 discloses a sprayable composition comprising a drug, for example ketoprofen, and the excipients: polyvinyl alcohol, polyvinylmethylether/maleic anhydride copolymer, glycerol, propylene glycol, ethanol and water.

The present invention provides topical dosage forms of NSAIDs that can be applied as a spray or as an aerosol.

### SUMMARY OF INVENTION

A sprayable analgesic preparation contains a nonsteroidal anti-inflammatory drug (NSAID) as defined in the claims
together with lauryl lactate, lactic acid, and glyceryl monolaurate dissolved
in a mixture of water and ethanol. The obtained aqueous ethanolic solution is useful for extended pain relief.

Optionally, the aqueous ethanolic solution can contain propylene glycol, a non-ionic surfactant having a HLB value of at least 12, and a thickener such as cellulose ethers, cross-linked alkyl acrylates, and the like.
The NSAIDs are the propionic acid derivatives selected from ketoprofen, ibuprofen, naproxen, and pharmaceutically acceptable salts thereof, as well as the acetic acid derivatives selected from diclofenac, indomethacin, etodolac, and pharmaceutically acceptable salts thereof.

### BRIEF DESCRIPTION OF DRAWINGS

In the drawings,
FIGURE 1 is a bar graph showing skin permeation by ketoprofen, applied as spray compositions, at selected time intervals after application, and comparison with a commercially available, ketoprofen-containing topical gel (Profenid® Sanofi-Aventis; 2.5% ketoprofen);
FIGURE 2 is a bar graph showing skin permeation by naproxen, applied as spray compositions, at selected time intervals after application, and comparison with a commercially available naproxen-containing topical gel (Naprosyn®, Syntex, 10% naproxen free acid);
FIGURE 3 is a bar graph showing skin permeation by ibuprofen, applied as a spray composition, at selected time intervals after application, and comparison with a commercially available ibuprofen-containing topical gel (Ibuleve®, DDD ltd., 5% ibuprofen);
FIGURE 4 is a bar graph showing skin permeation by diclofenac, and pharmaceutically acceptable salts thereof, applied as spray compositions, and comparison with a commercially available diclofenac sodium gel (Swiss Relief™, Mika Pharma GmbH, Fug, Switzerland, 4% diclofenac sodium);
FIGURE 5 is a bar graph showing the effect of propylene glycol on skin permeation in ibuprofen-containing spray compositions, and comparison with a commercially available ibuprofen-containing topical gel (Ibuleve®, DDD ltd., 5% ibuprofen);
FIGURE 6 is a bar graph showing skin permeation by ketoprofen with Brij 58 as a permeation enhancer, applied as spray compositions, at selected time intervals after application, and comparison with a commercially available, ketoprofen-containing topical gel (Profenid®, Sanofi-Aventis; 2.5% ketoprofen);
FIGURE 7 is a bar graph showing skin permeation by ketoprofen with propylene glycol laurate as a permeation enhancer, applied as spray compositions, at selected time intervals after application, and comparison with a commercially available, ketoprofen-containing topical gel (Profenid®, Sanofi-Aventis; 2.5% ketoprofen);
FIGURE 8 is a bar graph showing skin permeation by ketoprofen with propylene glycol caprylate as a permeation enhancer, applied as spray compositions, at selected time intervals after application, and comparison with a commercially available, ketoprofen-containing topical gel (Profenid®, Sanofi-Aventis; 2.5% ketoprofen);
FIGURE 9 is a bar graph showing skin permeation by ketoprofen with Sorbitan monolaurate as a permeation enhancer, applied as spray compositions, at selected time intervals after application, and comparison with a commercially available, ketoprofen-containing topical gel (Profenid®, Sanofi-Aventis; 2.5% ketoprofen);
FIGURE 10 is a bar graph showing skin permeation by ketoprofen with various Brij derivatives as a permeation enhancer, applied as spray compositions, at selected time intervals after application, and comparison with a commercially available, ketoprofen-containing topical gel (Profenid®, Sanofi-Aventis; 2.5% ketoprofen);
FIGURE 11 is a bar graph showing skin permeation by ketoprofen, applied as a spray composition after 3 months stored at 25°C and 40°C, at selected time intervals after application, and comparison with a commercially available, ketoprofen-containing topical gel (Profenid®, Sanofi-Aventis; 2.5% ketoprofen);
FIGURE 12 is a bar graph showing skin permeation by naproxen using 5% Naproxen sodium salt and Brij 58, applied as spray compositions, at selected time intervals after application, and comparison with a commercially available naproxen-containing topical gel (Naprosyn®, Syntex, 10% naproxen free acid);
FIGURE 13 is a bar graph showing skin permeation by naproxen using 2.5% Naproxen sodium salt and Brij 58, applied as spray compositions, at selected time intervals after application, and comparison with a commercially available naproxen-containing topical gel (Naprosyn®, Syntex, 10% naproxen free acid);
FIGURE 14 is a bar graph showing skin permeation by ibuprofen with and without isopropyl myristate (IPM), applied as spray compositions, at selected time intervals after application, and comparison with a commercially available ibuprofen-containing topical gel (Ibuleve®, DDD ltd., 5% ibuprofen);
FIGURE 15 is a bar graph showing skin permeation by ibuprofen with isopropyl myristate (IPM) and Brij 58, applied as spray compositions, at selected time intervals after application, and comparison with a commercially available ibuprofen-containing topical gel (Ibuleve®, DDD ltd., 5% ibuprofen);
FIGURE 16 is a bar graph showing skin permeation by diclofenac, and pharmaceutically acceptable salts thereof, applied as cream compositions, and comparison with a commercially available diclofenac sodium gel (Voltaren®, Novartis Pharma Productions GmbH, Wehr, Germany, 1% diclofenac sodium);
FIGURE 17 is a bar graph showing skin permeation by diclofenac, and pharmaceutically acceptable salts thereof, applied as spray compositions, and comparison with a commercially available diclofenac sodium gel (Swiss Relief™, Mika Pharma GmbH, Fug, Switzerland, 4% diclofenac sodium);
FIGURE 18 is a bar graph showing skin permeation by diclofenac sodium with Brij 58, and different levels of propylene glycol and lactic acid, applied as spray compositions, and comparison with a commercially available diclofenac sodium gel (Voltaren®, Novartis Pharma Productions GmbH, Wehr, Germany, 1% diclofenac sodium);
FIGURE 19 is a bar graph showing skin permeation by diclofenac diethylamine with Brij 58, and different levels of propylene glycol and lactic acid, applied as spray compositions, and comparison with a commercially available diclofenac sodium gel (Voltaren®, Novartis Pharma Productions GmbH, Wehr, Germany, 1% diclofenac sodium); and
FIGURE 20 is a bar graph showing skin permeation by ketoprofen compositions containing different levels of propylene glycol and hydroxypropyl cellulose thickeners.

### DESCRIPTION OF PREFERRED EMBODIMENTS

The present topical compositions are clear, sprayable, aqueous ethanolic solutions that contain dissolved therein a nonsteroidal anti-inflammatory drug
(NSAID) wherein the drug is either a propionic acid derivative selected from the group consisting of ketoprofen, ibuprofen, naproxen, and pharmaceutically acceptable salts thereof, or an acetic acid derivative selected from the group consisting of diclofenac, indomethacin, etodolac, and pharmaceutically acceptable salts thereof.

Illustrative NSAID salts suitable for use as active ingredients in the spray compositions are pharmaceutically acceptable salts of the aforementioned acetic acid derivatives, e.g., indomethacin salts such as indomethacin sodium, indomethacin meglumine, and the like, diclofenac salts such as diclofenac sodium, diclofenac diethylamine, diclofenac epolamine, and the like, as well as pharmaceutically acceptable salts of the aforementioned propionic acid derivatives, e.g., ibuprofen salts such as ibuprofen lysine, ibuprofen methylglucamine, and the like, naproxen salts such as naproxen piperazine, naproxen sodium, and the like.

The aqueous ethanolic solutions preferably contain the NSAID in an amount in the range of about 1 to about 10 percent by weight preferably about 5 percent by weight, based on the total weight of the solution.

Also present in the solutions is lauryl lactate (C₁₅H₃₀O₃), the ester of lauryl alcohol and lactic acid having the formula

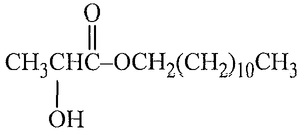

Preferably, lauryl lactate is present in the solution in an amount in the range of about 1 to about 5 weight percent, more preferably about 3 weight percent, based on the total weight of the solution.

The aqueous ethanolic solution also contains lactic acid (C₃H₆O₃; 2-hydroxypropanoic acid), preferably in an amount in the range of about 0.5 to about 5 weight percent, more preferably about 1.5 weight percent, based on the total weight of the solution; glyceryl monolaurate (C₁₅H₃₀O₄; dodecanoic acid 2,3-dihydroxypropyl ester), preferably in an amount in the range of about 2 to about 5 weight percent, more preferably about 3 weight percent, based on the total weight of the solution. Optionally, propylene glycol (C₃H₈O₂; propane- 1,2-diol) can be present, preferably in an amount in the range of about 5 to about 30 weight percent, more preferably about 10 weight percent, based on the total weight of the solution.

The remainder of the solution is constituted by water and ethanol, preferably present in a respective weight ratio of about 0.3:1 to about 2.6:1, more preferably in a respective weight ratio of about 1:1.

The aqueous ethanolic solution can also contain, as an optional ingredient, a non-ionic surfactant having a Hydrophile-Lipophile balance (HLB) value of at least 12. Preferred non-ionic surfactants are the alkoxylated alcohols. Particularly preferred is polyethylene glycol ether of cetyl alcohol represented by the formula CH₃(CH₂)₁₄CH₂(OCH₂CH₂)ₙOH, where n has an average of 10, and having a HLB value of about 15.7.

The foregoing clear aqueous ethanolic solutions are prepared by first combining the NSAID with lauryl lactate, lactic acid, and glyceryl monolaurate and thereafter dissolving the obtained admixture by gradual addition, at ambient temperature, of propylene glycol followed by the addition of alternating aliquots of water and ethanol. The non-ionic surfactant, if desired, is added to the admixture prior to the addition of water and ethanol.

Skin permeation studies of illustrative compositions embodying the invention were performed using dermatoned human female (46 years old) cadaver skin pieces from the back (Science Care, Aurora, CO; 250 Micrometers thick) Franz cells (3.65 ml volume, 0.55 cm² surface area) at 35° C. using heating/stirring blocks. Receptor compartment contained saline with sodium azide (pH 7.4). Two or three replicates (25 µl and control 25 mg) were made for each solution. Sampling volume was 300 µl. Fresh buffer was replaced after each sample removal. Sampling was carried out at 2, 4, 6 and 24 hours. The samples were assayed using high performance liquid chromatography (HPLC).

Respective controls were NSAID containing gels: Profenid® gel (2.5% ketoprofen; Sanofi Aventis, France), Ibuleve® gel (5% ibuprofen; DDD Ltd., UK), Naprosyn® gel (10% naproxen free acid; Syntex, Turkey), Swiss Relief™ Spray Gel (4% diclofenac sodium) Mika Pharma GmbH, Switzerland), and Voltaren® gel (1% diclofenac sodium) Novartis Pharma Productions GmbH, Wehr, Germany.

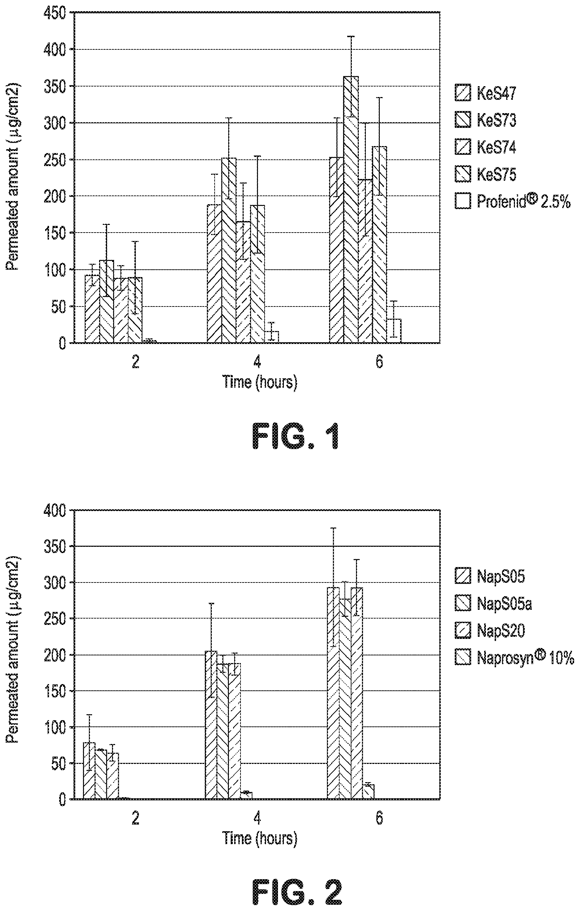

The experimental results obtained with a ketoprofen spray composition are presented in Tables 1 and 2 below, and in FIGURE 1.

**TABLE 1**

| Ketoprofen Spray Composition | | | | |
|---|---|---|---|---|
| | Composition, wt.% | | | |
| Ingredients | KeS47 | KeS73 | KeS74 | KeS75 |
| Ketoprofen | 5 | 5 | 5 | 5 |
| Propylene glycol | 10 | 10 | 10 | 10 |
| Lauryl lactate | 3 | 3 | 3 | 3 |
| Lactic acid | 1.5 | 1.5 | 1.5 | 1.5 |
| Ceteth-20¹ | | 3 | | |
| Imwitor 412² | | | 3 | |
| Capmul PG-8³ | | | | 3 |
| Glyceryl monolaurate | 3 | 3 | 3 | 3 |
| Ethanol | 37.5 | 27.25 | 39.5 | 34.5 |
| Water | 40 | 47.25 | 35 | 40 |
| TOTAL | 100 | 100 | 100 | 100 |

| | | | | |
|---|---|---|---|---|
| ¹ CH₃(CH₂)₁₄CH₂(OCH₂CH₂)ₙOH, n average value 20; HLB 15.7; also Brij 58 ² Propylene glycol laurate, HLB 4-5 ³ Propylene glycol monocaprylate, HLB 5-6 | | | | |

**TABLE 2**

| Permeation Data | | | | | | | | | | |
|---|---|---|---|---|---|---|---|---|---|---|
| | Cumulative Amount in Receptor, µg/cm² | | | | | | | | | |
| Time, hours | KeS47 | ±SD | KeS73 | ±SD | KeS74 | ±SD | KeS75 | ±SD | Profenid 2.5% | ±SD |
| 2 | 92.35 | 14.49 | 112.54 | 49.36 | 88.47 | 16.40 | 88.95 | 49.36 | 2.93 | 5.93 |
| 4 | 189.43 | 40.72 | 252.20 | 54.87 | 166.05 | 52.11 | 188.46 | 65.71 | 15.74 | 21.11 |
| 6 | 253.32 | 53.34 | 363.38 | 54.64 | 222.65 | 77.00 | 267.87 | 66.92 | 32.57 | 33.53 |

The above data show that spray compositions provided better skin permeation for ketoprofen than a ketoprofen containing gel composition, and that skin permeation could be further enhanced by an alkoxylated alcohol having an HLB value of 15.7.

The experimental results obtained with a naproxen spray composition are presented in Table 3 and 4 below, and in FIGURE 2.

**TABLE 3**

| Naproxen Spray Composition | | | | |
|---|---|---|---|---|
| | Composition, wt.% | | | |
| Ingredients | NapS05 | NapS05a | NapS20 | Naprosyn® |
| Naproxen | | | | 10 |
| Naproxen Na | 5 | 4.7 | 5 | |
| Propylene glycol | 10 | 9.5 | 10 | |
| Lauryl lactate | 3 | 2.8 | 3 | |
| Isopropyl myristate | | | 3 | |
| Lactic acid | 1.5 | 1.4 | 1.5 | |
| Ceteth-20¹ | | 2.8 | 3 | |
| Glyceryl monolaurate | 3 | 2.8 | 3 | |
| Ethanol | 55.5 | 42.7 | 41.5 | |
| Water | 22 | 33.2 | 30 | |
| TOTAL | 100 | 100 | 100 | |

**TABLE 4**

| Naproxen Permeation Data | | | | | | | | |
|---|---|---|---|---|---|---|---|---|
| Time, hours | Cumulative Amount in Receptor, µg/cm² | | | | | | | |
| | NapS05 | ±SD | NapS05a | ±SD | NapS20 | ±SD | Naprosyn® | ±SD |
| 2 | 78.92 | 37.94 | 68.91 | 0.54 | 64.42 | 11.20 | 1.65 | 0.28 |
| 4 | 206.15 | 65.01 | 187.63 | 11.82 | 187.47 | 15.16 | 9.67 | 1.17 |
| 6 | 293.37 | 81.72 | 277.23 | 23.83 | 292.88 | 38.90 | 20.38 | 2.42 |

The above data show that naproxen containing spray compositions provided better skin permeation for naproxen than the Naprosyn® 10% naproxen gel. The incorporation of higher levels of water did not reduce the permeation of naproxen.

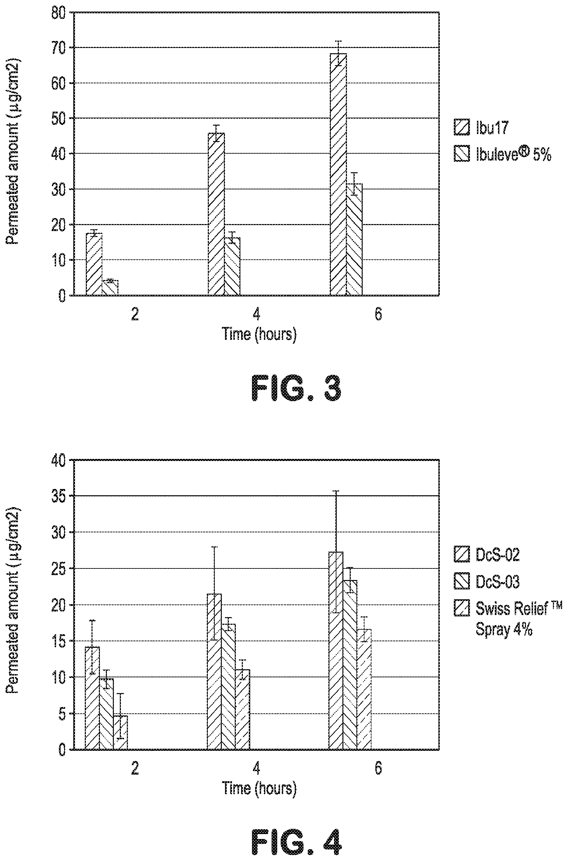

The experimental results obtained with an ibuprofen spray composition are presented in Tables 5 and 6 below, and in FIGURE 3. The experimental procedure was the same as that for the ketoprofen and naproxen spray compositions, except that the dermatomed cadaver skin was that of a human male, 72 years old.

**TABLE 5**

| Ibuprofen Spray Composition | | |
|---|---|---|
| | Composition, wt.% | |
| Ingredients | Ibu17 | Ibuleve® |
| Ibuprofen | 5 | 5 |
| Lauryl lactate | 3 | |
| Lactic acid | 1.5 | |
| Glyceryl monolaurate | 3 | |
| Ethanol | 47.5 | |
| Water | 40 | |
| TOTAL | 100 | |

**TABLE 6**

| Ibuprofen Permeation Data | | | | |
|---|---|---|---|---|
| Time, hours | Cumulative Amount in Receptor, µg/cm² | | | |
| | Ibu17 | ±SD | Ibuleve® | ±SD |
| 2 | 17.59 | 0.96 | 4.12 | 1.22 |
| 4 | 45.81 | 0.75 | 16.31 | 0.45 |
| 6 | 68.39 | 0.16 | 31.47 | 0.32 |

The above data show that an ibuprofen containing spray composition provided better skin permeation for ibuprofen than the Ibuleve® ibuprofen gel.

The experimental results obtained with a diclofenac spray composition are presented in Tables 7 and 8 below, and in FIGURE 4. The experimental procedure was the same as that for the ketoprofen and naproxen spray compositions except that the dermatomed cadaver skin was that of a 79-year old human male.

**TABLE 7**

| Diclofenac Spray Composition | | | |
|---|---|---|---|
| | Composition, wt.% | | |
| Ingredients | DcS02 | DcS03 | Swiss Relief™ |
| Diclofenac Na | | | 4⁴ |
| Diclofenac diethylamine | | 1 | |
| Propylene glycol | 10 | 10 | |
| Lauryl lactate | 3 | 3 | |
| Lactic acid | 1.5 | 1.5 | |
| Glyceryl monolaurate | 3 | 3 | |
| Ethanol | 48.5 | 48.5 | |
| Water | 33 | 33 | |
| TOTAL | 100 | 100 | |

| | | | |
|---|---|---|---|
| ⁴ Swiss Relief™ spray gel contains 4 wt. % diclofenac sodium together with inactive ingredients isopropyl alcohol, soy bean lecithin, ethanol, disodium phosphate dodecahydrate, sodium dihodrogen phosphate dehydrate, sodium edetate, propylene glycol, peppermint oil, ascorbyl palmitate, hydrochloric acid (10% w/w), sodium hydroxide (10% w/w), purified water. | | | |

**TABLE 8**

| Diclofenac Permeation Data | | | | | | |
|---|---|---|---|---|---|---|
| Time, hours | Cumulative Amount in Receptor, µg/cm² | | | | | |
| | DcS-02 | ±SD | DcS-03 | ±SD | Swiss Relief™ spray gel | ±SD |
| 2 | 14.16 | 3.67 | 9.71 | 1.25 | 4.64 | 3.11 |
| 4 | 21.51 | 6.42 | 17.30 | 0.93 | 11.08 | 1.33 |
| 6 | 27.31 | 8.43 | 23.39 | 1.76 | 16.62 | 1.70 |

The above data show that a diclofenac containing spray composition provided better skin permeation for diclofenac than a spray gel composition that has a relatively larger concentration of diclofenac.

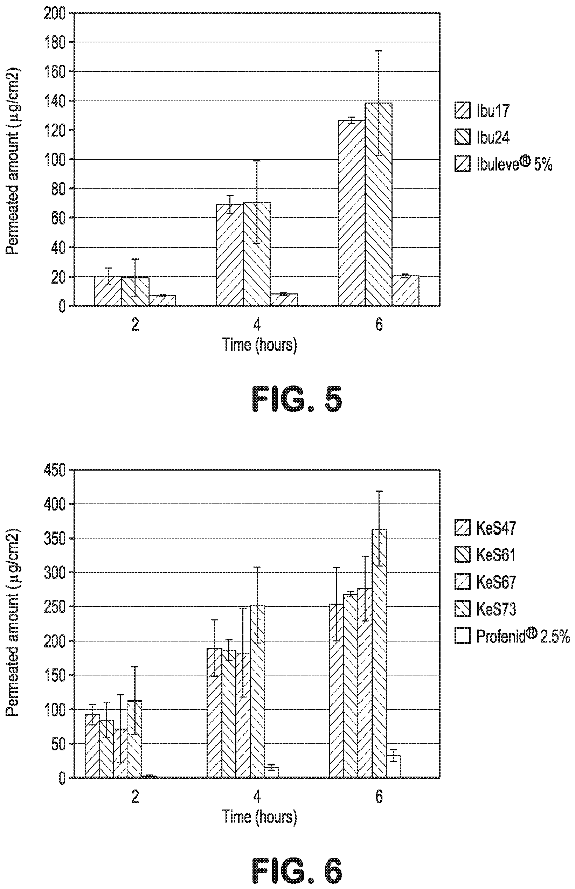

The effect of propylene glycol in an ibuprofen spray composition was investigated using cadaver skin from a 72 year-old human male. The experimental results are presented in Tables 9 and 10 below, and in FIGURE 5.

**TABLE 9**

| Ibuprofen Spray Compositions | | | |
|---|---|---|---|
| | Composition, wt.% | | |
| Ingredients | Ibu17 | Ibu24 | Ibuleve® |
| Ibuprofen | 5 | 5 | 5 |
| Propylene glycol | | 10 | |
| Lauryl lactate | 3 | 3 | |
| Lactic acid | 1.5 | 1.5 | |
| Glyceryl monolaurate | 3 | 3 | |
| Ethanol | 47.5 | 37.5 | |
| Water | 40 | 40 | |
| TOTAL | 100 | 100 | |

**TABLE 10**

| Ibuprofen Permeation Data | | | | | | |
|---|---|---|---|---|---|---|
| Time, hours | Cumulative Amount in Receptor, µg/cm² | | | | | |
| | Ibu17 | ±SD | Ibu24 | ±SD | Ibuleve® | ±SD |
| 2 | 20.22 | 5.59 | 19.36 | 12.52 | 7.17 | 12.42 |
| 4 | 69.45 | 5.95 | 71.08 | 27.82 | 8.65 | 1.80 |
| 6 | 127.06 | 2.01 | 138.70 | 35.68 | 20.97 | 8.20 |

The above data show that propylene glycol in the spray composition enhanced the skin penetration of ibuprofen.

The experimental results obtained with a ketoprofen spray composition and a nonionic surfactant, polyoxyethylene (20) cetyl ether (Brij 58), as a permeation enhancer are presented in Tables 11 and 12 below, and in FIGURE 6.

**TABLE 11**

| Ketoprofen and a Nonionic Surfactant Spray Composition | | | | | |
|---|---|---|---|---|---|
| The compositions KeS61 and KeS67 are not in the scope of the claims. | | | | | |
| | Composition, wt.% | | | | |
| Ingredients | KeS47 | KeS61 | KeS67 | KeS73 | KeS73a |
| Ketoprofen | 5 | 5 | 5 | 5 | 5 |
| Propylene glycol | 10 | 10 | 10 | 10 | 10 |
| Lauryl lactate | 3 | 3 | 0 | 3 | 3 |
| Lactic acid | 1.5 | 1.5 | 1.5 | 1.5 | 1.5 |
| Brij 58¹ | | 3 | 3 | 3 | 3 |
| Glyceryl monolaurate | 3 | 0 | 3 | 3 | 3 |
| Ethanol | 37.5 | 37.5 | 37.5 | 27.25 | 25.25 |
| Water | 40 | 40 | 40 | 47.25 | 49.25 |
| TOTAL | 100 | 100 | 100 | 100 | 100 |

**TABLE 12**

| Permeation Data | | | | | | | | | | |
|---|---|---|---|---|---|---|---|---|---|---|
| Time, hours | Cumulative Amount in Receptor, µg/cm² | | | | | | | | | |
| | KeS47 | ±SD | KeS61 | ±SD | KeS67 | ±SD | KeS73 | ±SD | Profenid 2.5% | ±SD |
| 2 | 92.35 | 14.49 | 84.55 | 25.06 | 71.71 | 49.49 | 112.54 | 49.36 | 15.60 | 5.93 |
| 4 | 189.43 | 40.72 | 186.97 | 15.02 | 182.54 | 64.55 | 252.20 | 54.87 | 70.75 | 21.11 |
| 6 | 253.32 | 53.34 | 267.88 | 3.95 | 275.99 | 47.09 | 363.38 | 54.64 | 134.62 | 33.53 |

The above data show that the addition of Brij 58 helped to increase water levels. All formulations exhibited similar permeation behavior; however, KeS73 showed slightly higher permeation. KeS73a was slightly cloudy.

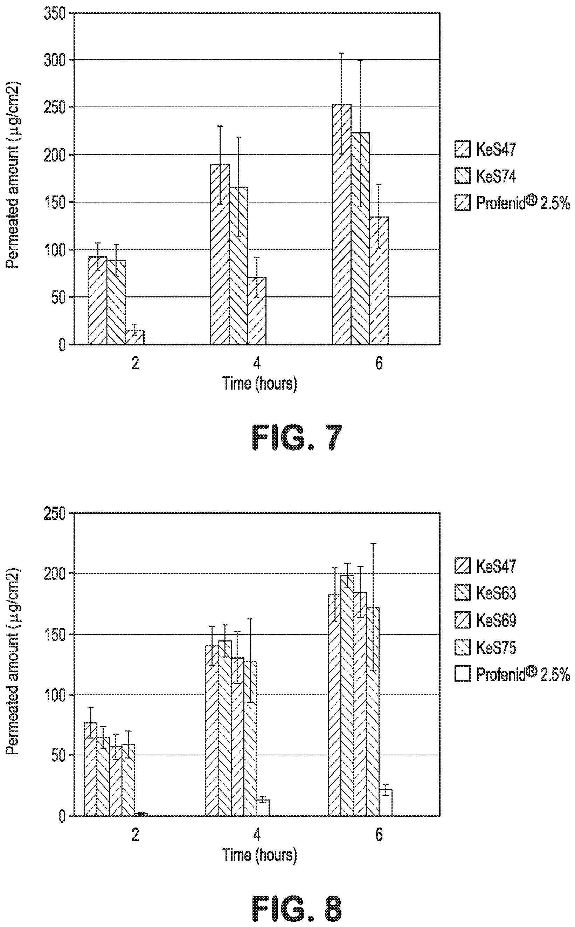

The experimental results obtained with a ketoprofen spray composition and propylene glycol laurate as a permeation enhancer are presented in Tables 13 and 14 below, and in FIGURE 7.

**TABLE 13**

| Ketoprofen and Propylene Glycol Laurate Spray Composition | | | | |
|---|---|---|---|---|
| The compositions KeS62 and KeS68 are not in the scope of the claims. | | | | |
| Ingredients | Composition, wt.% | | | |
| | KeS47 | KeS62 | KeS68 | KeS74 |
| Ketoprofen | 5 | 5 | 5 | 5 |
| Propylene glycol | 10 | 10 | 10 | 10 |
| Lauryl lactate | 3 | 3 | 0 | 3 |
| Lactic acid | 1.5 | 1.5 | 1.5 | 1.5 |
| Propylene glycol laurate | | 3 | 3 | 3 |
| Glyceryl monolaurate | 3 | 0 | 3 | 3 |
| Ethanol | 37.5 | 42.5 | 42.5 | 39.5 |
| Water | 40 | 35 | 35 | 35 |
| TOTAL | 100 | 100 | 100 | 100 |

**TABLE 14**

| Permeation Data | | | | | | |
|---|---|---|---|---|---|---|
| Time, hours | Cumulative Amount in Receptor, µg/cm² | | | | | |
| | KeS47 | ±SD | KeS74 | ±SD | Profenid 2.5% | ±SD |
| 2 | 92.35 | 14.49 | 88.47 | 16.40 | 15.60 | 5.93 |
| 4 | 189.43 | 40.72 | 166.05 | 52.11 | 70.75 | 21.11 |
| 6 | 253.32 | 53.34 | 222.65 | 77.00 | 134.62 | 33.53 |

Only formulations KeS47 and KeS74 gave clear solutions. The above data show that the KeS47 and KeS74 formulations exhibited nearly the same permeation behavior.

The experimental results obtained with a ketoprofen spray composition and propylene glycol caprylate as a permeation enhancer are presented in Tables 15 and 16 below, and in FIGURE 8. The experimental procedure was the same as that for the previous ketoprofen spray compositions, except that the dermatomed cadaver skin was that of a human male, 79 years old.

**TABLE 15**

| Ketoprofen and Propylene Glycol Caprylate Spray Composition | | | | |
|---|---|---|---|---|
| The compositions KeS63 and KeS69 are not in the scope of the claims. | | | | |
| | Composition, wt.% | | | |
| Ingredients | KeS47 | KeS63 | KeS69 | KeS75 |
| Ketoprofen | 5 | 5 | 5 | 5 |
| Propylene glycol | 10 | 10 | 10 | 10 |
| Lauryl lactate | 3 | 3 | 0 | 3 |
| Lactic acid | 1.5 | 1.5 | 1.5 | 1.5 |
| Propylene glycol caprylate | | 3 | 3 | 3 |
| Glyceryl monolaurate | 3 | 0 | 3 | 3 |
| Ethanol | 37.5 | 37.5 | 37.5 | 34.5 |
| Water | 40 | 40 | 40 | 40 |
| TOTAL | 100 | 100 | 100 | 100 |

**TABLE 16**

| Permeation Data | | | | | | | | | | |
|---|---|---|---|---|---|---|---|---|---|---|
| Time, hours | Cumulative Amount in Receptor, µg/cm² | | | | | | | | | |
| | KeS47 | ±SD | KeS63 | ±SD | KeS69 | ±SD | KeS75 | ±SD | Profenid 2.5% | ±SD |
| 2 | 77.12 | 12.81 | 64.90 | 9.02 | 57.22 | 10.33 | 58.98 | 10.81 | 2.27 | 0.44 |
| 4 | 140.68 | 16.03 | 144.77 | 13.15 | 131.23 | 21.40 | 128.25 | 34.31 | 13.60 | 2.05 |
| 6 | 183.17 | 22.46 | 198.59 | 10.20 | 184.99 | 21.11 | 172.43 | 52.30 | 21.69 | 4.50 |

The above data show that all of the formulations exhibited comparable permeation behavior.

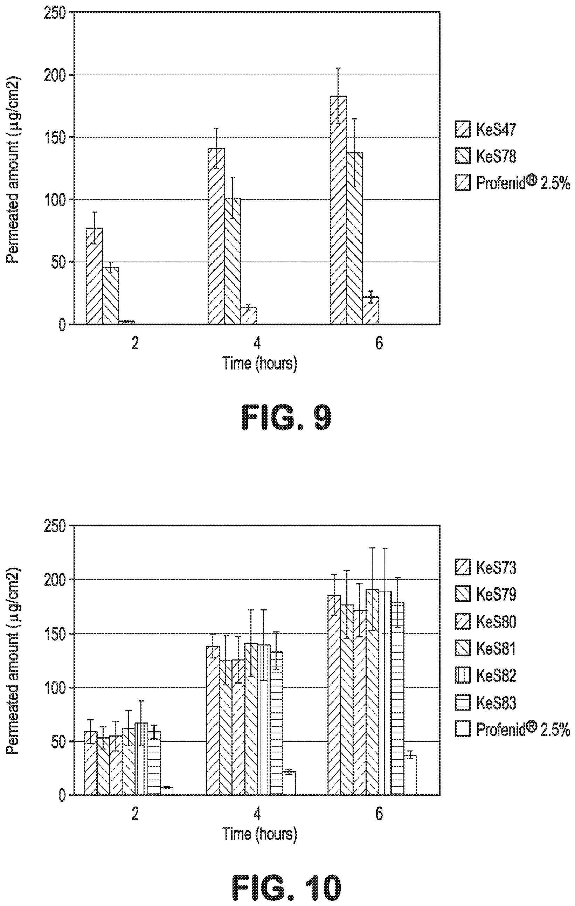

The experimental results obtained with a ketoprofen spray composition and Sorbitan monolaurate as a permeation enhancer are presented in Tables 17 and 18 below, and in FIGURE 9. The experimental procedure was the same as that for the previous ketoprofen spray compositions, except that the dermatomed cadaver skin was that of a human male, 79 years old.

**TABLE 17**

| Ketoprofen and Sorbitan Monolaurate Spray Composition | | | | |
|---|---|---|---|---|
| The compositions KeS66 and KeS72 are not in the scope of the claims. | | | | |
| | Composition, wt.% | | | |
| Ingredients | KeS47 | KeS66 | KeS72 | KeS78 |
| Ketoprofen | 5 | 5 | 5 | 5 |
| Propylene glycol | 10 | 10 | 10 | 10 |
| Lauryl lactate | 3 | 3 | 0 | 3 |
| Lactic acid | 1.5 | 1.5 | 1.5 | 1.5 |
| Sorbitan monolaurate | | 3 | 3 | 3 |
| Glyceryl monolaurate | 3 | 0 | 3 | 3 |
| Ethanol | 37.5 | 57.5 | 57.5 | 34.5 |
| Water | 40 | 20 | 20 | 40 |
| TOTAL | 100 | 100 | 100 | 100 |

**TABLE 18**

| Permeation Data | | | | | | |
|---|---|---|---|---|---|---|
| Time, hours | Cumulative Amount in Receptor, µg/cm² | | | | | |
| | KeS47 | ±SD | KeS78 | ±SD | Profenid 2.5% | ±SD |
| 2 | 77.12 | 12.81 | 45.37 | 4.10 | 2.27 | 0.44 |
| 4 | 140.68 | 16.03 | 101.16 | 16.60 | 13.60 | 2.05 |
| 6 | 183.17 | 22.46 | 137.44 | 27.22 | 21.69 | 4.50 |

Only formulations KeS47 and KeS78 gave clear solutions. The above data show that permeation from KeS78 was slightly lower than KeS47.

The experimental results obtained with a ketoprofen spray composition and various Brij derivatives as a permeation enhancer are presented in Tables 19 and 20 below, and in FIGURE 10. The experimental procedure was the same as that for the previous ketoprofen spray compositions, except that the dermatomed cadaver skin was that of a human male, 79 years old.

**TABLE 19**

| Ketoprofen and Non-ionic Surfactant Spray Composition | | | | | | |
|---|---|---|---|---|---|---|
| | Composition, wt.% | | | | | |
| Ingredients | KeS73 | KeS79 | KeS80 | KeS81 | KeS82 | KeS83 |
| Ketoprofen | 5 | 5 | 5 | 5 | 5 | 5 |
| Propylene glycol | 10 | 10 | 10 | 10 | 10 | 10 |
| Lauryl lactate | 3 | 3 | 3 | 3 | 3 | 3 |
| Lactic acid | 1.5 | 1.5 | 1.5 | 1.5 | 1.5 | 1.5 |
| Brij 58¹ | 3 | | | | | |
| Brij 30⁵ | | 3 | | | | |
| Brij 35⁶ | | | 3 | | | |
| Brij 72⁷ | | | | 3 | | |
| Brij 98⁸ | | | | | 3 | |
| Brij 721⁹ | | | | | | 3 |
| Glyceryl monolaurate | 3 | 3 | 3 | 3 | 3 | 3 |
| Ethanol | 27.25 | 27.25 | 27.25 | 27.25 | 27.25 | 27.25 |
| Water | 47.25 | 47.25 | 47.25 | 47.25 | 47.25 | 47.25 |
| TOTAL | 100 | 100 | 100 | 100 | 100 | 100 |

| | | | | | | |
|---|---|---|---|---|---|---|
| ⁵ poly(oxyethylene)(4) lauryl ether ⁶ poly(oxyethylene)(23) lauryl ether ⁷ poly(oxyethylene)(2) stearyl ether ⁸ poly(oxyethylene)(20) oleyl ether ⁹ poly(oxyethylene)(21) stearyl ether | | | | | | |

**TABLE 20**

| Permeation Data | | | | | | | | | | | | | | |
|---|---|---|---|---|---|---|---|---|---|---|---|---|---|---|
| | Cumulative Amount in Receptor, µg/cm² | | | | | | | | | | | | | |
| Time, hours | KeS73 | ±SD | KeS79 | ±SD | KeS80 | ±SD | KeS81 | ±SD | KeS82 | ±SD | KeS83 | ±SD | Profenid 2.5% | ±SD |
| 2 | 58.92 | 11.11 | 53.39 | 10.39 | 54.79 | 13.75 | 61.91 | 16.23 | 67.06 | 20.54 | 59.12 | 6.21 | 7.15 | 0.42 |
| 4 | 138.70 | 11.05 | 125.49 | 22.77 | 125.93 | 21.40 | 141.07 | 30.91 | 139.71 | 32.62 | 134.15 | 17.36 | 21.66 | 1.94 |
| 6 | 185.84 | 18.67 | 176.90 | 31.52 | 171.52 | 24.45 | 191.21 | 38.25 | 189.49 | 39.15 | 178.84 | 23.19 | 37.41 | 3.53 |

The above data show that all formulations containing the non-ionic surfactants permitted higher water content while showing similar behavior with respect to permeation.

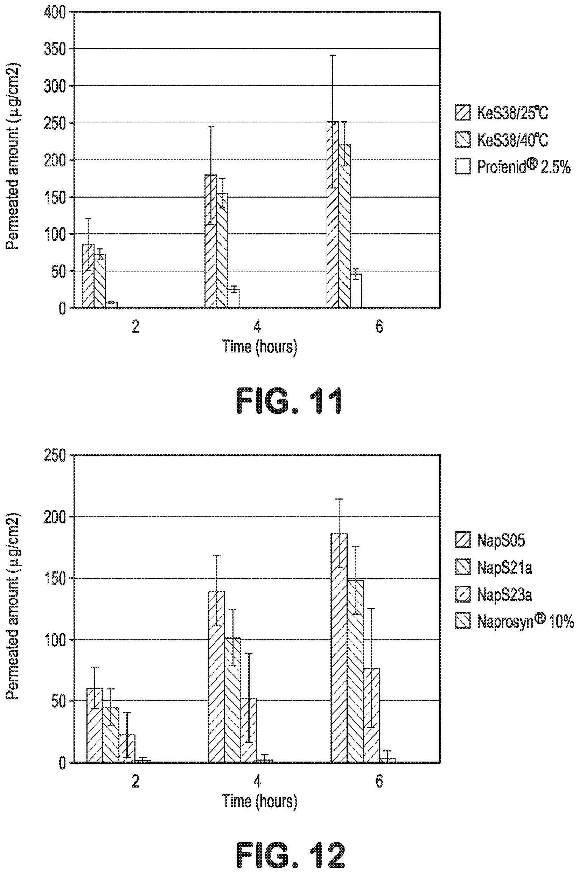

The experimental results showing permeation of a ketoprofen spray composition after storage for three (3) months at 25°C and 40°C is presented in Tables 21 and 22 below, and in FIGURE 11. The experimental procedure was the same as that for the previous ketoprofen spray compositions, except that the dermatomed cadaver skin was from the thigh of a human male, 79 years old.

**TABLE 21**

| Ketoprofen Spray Composition | | |
|---|---|---|
| | Composition, wt.% | |
| Ingredients | KeS38/25°C | KeS38/40°C |
| Ketoprofen | 5 | 5 |
| Lauryl lactate | 3 | 3 |
| Lactic acid | 1.5 | 1.5 |
| Glyceryl monolaurate | 3 | 3 |
| Ethanol | 47.5 | 47.25 |
| Water | 40 | 40 |
| TOTAL | 100 | 100 |

**TABLE 22**

| Permeation Data | | | | | | |
|---|---|---|---|---|---|---|
| | Cumulative Amount in Receptor, µg/cm² | | | | | |
| Time, hours | KeS38/25°C | ±SD | KeS38/40°C | ±SD | Profenid 2.5% | ±SD |
| 2 | 85.61 | 35.14 | 72.35 | 7.44 | 6.97 | 1.23 |
| 4 | 178.97 | 66.42 | 154.72 | 19.61 | 25.34 | 3.96 |
| 6 | 251.24 | 89.44 | 220.46 | 29.64 | 45.35 | 6.86 |

The above data show that both formulations exhibited similar permeation behavior after three months of storage.

The experimental results obtained with a naproxen spray composition using 5% Naproxen sodium and Brij 58 are presented in Table 23 and 24 below, and in FIGURE 12. The experimental procedure was the same as that for the previous spray compositions, except that the dermatomed cadaver skin was from the thigh of a human male, 79 years old.

**TABLE 23**

| Naproxen Spray Composition Using 5% Naproxen Sodium and a Non-ionic Surfactant | | | | | |
|---|---|---|---|---|---|
| | Composition, wt.% | | | | |
| Ingredients | NapS05 | NapS21a | NapS22a | NapS23a | NapS24 |
| Naproxen | 0 | 0 | 0 | 0 | 0 |
| Naproxen Na | 5 | 5 | 5 | 5 | 5 |
| Propylene glycol | 10 | 10 | 10 | 10 | 10 |
| Lauryl lactate | 3 | 3 | 3 | 3 | 3 |
| Lactic acid | 1.5 | 0.5 | 3 | 0.5 | 3 |
| Brij 58¹ | | 0 | 0 | 3 | 3 |
| Glyceryl monolaurate | 3 | 3 | 3 | 3 | 3 |
| Ethanol | 55.5 | 34.5 | 46 | 30 | 47 |
| Water | 22 | 44 | 30 | 45.5 | 26 |
| TOTAL | 100 | 100 | 100 | 100 | 100 |

**TABLE 24**

| Naproxen Permeation Data | | | | | | | | |
|---|---|---|---|---|---|---|---|---|
| | Cumulative Amount in Receptor, µg/cm² | | | | | | | |
| Time, hours | NapS05 | ±SD | NapS21a | ±SD | NapS23a | ±SD | Naprosyn® | ±SD |
| 2 | 60.77 | 16.57 | 44.92 | 14.84 | 22.70 | 18.23 | 1.67 | 2.90 |
| 4 | 139.90 | 28.22 | 101.66 | 22.94 | 52.91 | 36.06 | 2.37 | 4.10 |
| 6 | 186.06 | 27.93 | 147.81 | 27.39 | 77.01 | 48.09 | 3.61 | 6.25 |

The above data show that by decreasing the level of water and lactic acid, formulations with higher levels of ethanol were prepared. Reduction in lactic acid and addition of Brij 58 resulted in lower skin permeation. A precipitate was noted in NapS22a and NapS24.

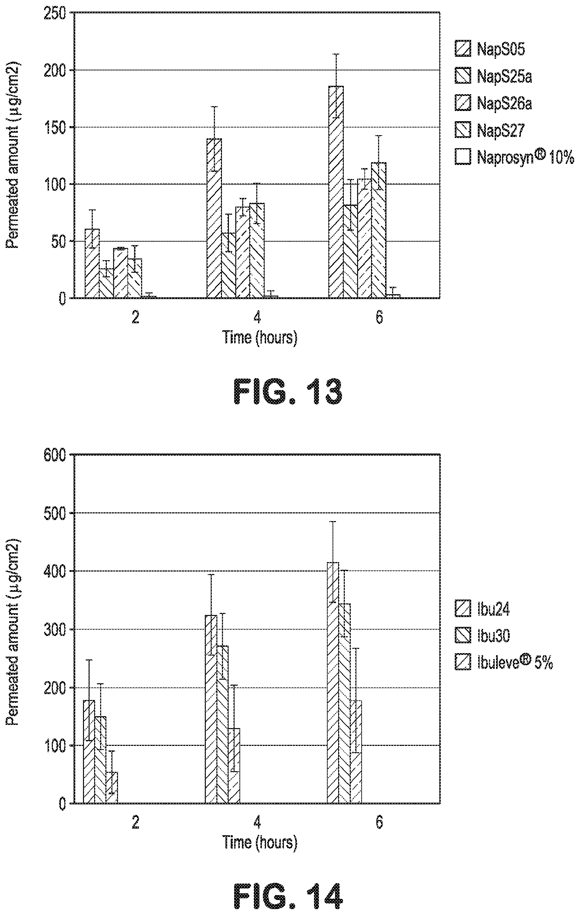

The experimental results obtained with a naproxen spray composition using 2.5% Naproxen sodium and Brij 58 are presented in Table 25 and 26 below, and in FIGURE 13. The experimental procedure was the same as that for the previous spray compositions, except that the dermatomed cadaver skin was from the thigh of a human male, 79 years old.

**TABLE 25**

| Naproxen Spray Composition Using 2.5% Naproxen Sodium and a Non-ionic Surfactant | | | | | | | |
|---|---|---|---|---|---|---|---|
| | Composition, wt.% | | | | | | |
| Ingredients | NapS05 | NapS25 | NapS25a | NapS26 | NapS26a | NapS27 | NapS28 |
| Naproxen | 0 | 0 | 0 | 0 | 0 | 0 | 0 |
| Naproxen Na | 5 | 2.5 | 2.5 | 2.5 | 2.5 | 2.5 | 2.5 |
| Propylene glycol | 10 | 10 | 10 | 10 | 10 | 10 | 10 |
| Lauryl lactate | 3 | 3 | 3 | 3 | 3 | 3 | 3 |
| Lactic acid | 1.5 | 0.5 | 0.5 | 1.5 | 1.5 | 0.5 | 1.5 |
| Brij 58¹ | | 3 | 3 | 3 | 3 | 0 | 0 |
| Glyceryl monolaurate | 3 | 3 | 3 | 3 | 3 | 3 | 3 |
| Ethanol | 55.5 | 22 | 32 | 37 | 40 | 36 | 45 |
| Water | 22 | 56 | 46 | 40 | 37 | 45 | 35 |
| TOTAL | 100 | 100 | 100 | 100 | 100 | 100 | 100 |

A precipitate was noted in NapS05, NapS25, NapS26 and NapS28.

**TABLE 26**

| Naproxen Permeation Data | | | | | | | | | | |
|---|---|---|---|---|---|---|---|---|---|---|
| | Cumulative Amount in Receptor, µg/cm² | | | | | | | | | |
| Time, hours | NapS05 | ±SD | NapS25a | ±SD | NapS26a | ±SD | NapS27 | ±SD | Naprosyn® | ±SD |
| 2 | 60.77 | 16.57 | 26.05 | 7.07 | 43.55 | 1.01 | 34.57 | 11.52 | 1.67 | 2.90 |
| 4 | 139.90 | 28.22 | 57.48 | 16.40 | 79.99 | 7.85 | 83.28 | 17.65 | 2.37 | 4.10 |
| 6 | 186.06 | 27.93 | 82.19 | 21.96 | 104.69 | 8.99 | 119.11 | 23.73 | 3.61 | 6.25 |

The above data show that reduction of Naproxen levels to 2.5% caused a significant reduction in skin permeation.

The effect of isopropyl myristate in an ibuprofen spray composition was investigated. The experimental results are presented in Tables 27 and 28 below, and in FIGURE 14. The experimental procedure was the same as that for the previous spray compositions, except that the dermatomed cadaver skin was from the thigh of a human male, 79 years old.

**TABLE 27**

| Ibuprofen with and without Isopropyl Myristate Spray Compositions | | |
|---|---|---|
| | Composition, wt.% | |
| Ingredients | Ibu24 | Ibu30 |
| Ibuprofen | 5 | 5 |
| Propylene glycol | 10 | 10 |
| Isopropyl myristate | 0 | 3 |
| Lauryl lactate | 3 | 3 |
| Lactic acid | 1.5 | 1.5 |
| Glyceryl monolaurate | 3 | 3 |
| Ethanol | 37.5 | 39.5 |
| Water | 40 | 35 |
| TOTAL | 100 | 100 |

**TABLE 28**

| Ibuprofen with and without Isopropyl Myristate Permeation Data | | | | | | |
|---|---|---|---|---|---|---|
| Time, hours | Cumulative Amount in Receptor, µg/cm² | | | | | |
| | Ibu24 | ±SD | Ibu30 | ±SD | Ibuleve® | ±SD |
| 2 | 177.88 | 34.92 | 149.67 | 43.10 | 53.86 | 36.32 |
| 4 | 324.99 | 58.07 | 271.38 | 62.99 | 129.82 | 74.30 |
| 6 | 415.42 | 62.34 | 344.02 | 61.75 | 177.62 | 89.78 |

The above data show that addition of isopropyl myristate in the spray composition did not further enhance the skin permeation of ibuprofen.

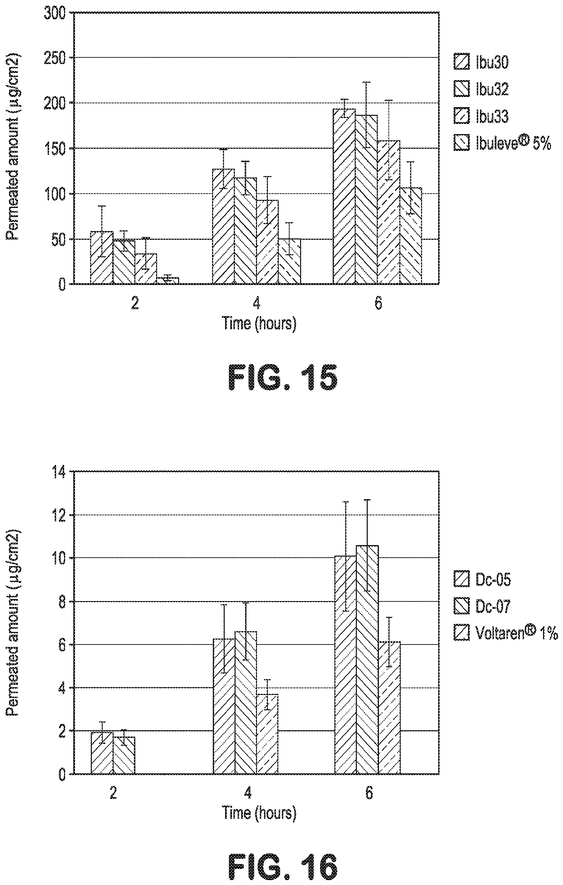

The effect of isopropyl myristate and Brij 58 in an ibuprofen spray composition was investigated. The experimental results are presented in Tables 29 and 30 below, and in FIGURE 15. The experimental procedure was the same as that for the previous spray compositions, except that the dermatomed cadaver skin was from the back of a human male, 46 years old.

**TABLE 29**

| Ibuprofen with Isopropyl Myristate and Brij 58 Spray Compositions | | | |
|---|---|---|---|
| | Composition, wt.% | | |
| Ingredients | Ibu30 | Ibu32 | Ibu33 |
| Ibuprofen | 5 | 5 | 5 |
| Propylene glycol | 10 | 10 | 10 |
| Isopropyl myristate | 3 | 0 | 3 |
| Lauryl lactate | 3 | 3 | 3 |
| Lactic acid | 1.5 | 1.5 | 1.5 |
| Brij 58¹ | | 3 | 3 |
| Glyceryl monolaurate | 3 | 3 | 3 |
| Ethanol | 39.5 | 28 | 31.5 |
| Water | 35 | 46.5 | 40 |
| TOTAL | 100 | 100 | 100 |

**TABLE 30**

| Ibuprofen Permeation Data | | | | | | | | |
|---|---|---|---|---|---|---|---|---|
| Time, hours | Cumulative Amount in Receptor, µg/cm² | | | | | | | |
| | Ibu30 | ±SD | Ibu32 | ±SD | Ibu33 | ±SD | Ibuleve® | ±SD |
| 2 | 58.28 | 27.72 | 47.79 | 10.56 | 33.70 | 17.05 | 7.06 | 3.27 |
| 4 | 127.23 | 21.44 | 117.41 | 18.49 | 93.08 | 25.68 | 50.52 | 17.68 |
| 6 | 193.89 | 9.97 | 187.01 | 36.12 | 159.15 | 43.60 | 106.53 | 28.78 |

The above data show that addition of Brij 58 helped to increase the level of water in the formulation; however, the addition of isopropyl myristate and Brij 58 in the spray composition did not further enhance the skin permeation of ibuprofen.

The effects of two types of diclofenac were investigated. The experimental results obtained with a diclofenac cream composition (which is not in the scope of the claims) using diclofenac sodium and diclofenac diethylamine are presented in Tables 31 and 32, below, and in FIGURE 16. The dermatomed cadaver skin was from the back of a human male, 79 years old.

**TABLE 31**

| Diclofenac Cream Composition | | |
|---|---|---|
| | Composition, wt.% | |
| Ingredients | Dc-05 | Dc-07 |
| Diclofenac Na | | |
| Diclofenac diethylamine | | 1 |
| Carbopol 980 NF¹⁰ | 0.5 | 0.5 |
| Ultrez 10¹¹ | 1 | 1 |
| Deionized water | 63.95 | 63.95 |
| Disodium EDTA | 0.05 | 0.05 |
| Isopropyl myristate | 3 | 3 |
| Ethanol | 10 | 10 |
| Propylene glycol | 10 | 10 |
| Isopropanol | 9 | 9 |
| Triethanolamine, NF | 1.5 | 1.5 |
| TOTAL | 100 | 100 |

| | | |
|---|---|---|
| ¹⁰ acrylic acid homopolymer ¹¹ cross-linked polyacrylic acid polymer | | |

**TABLE 32**

| Diclofenac Permeation Data | | | | | | |
|---|---|---|---|---|---|---|
| Time, hours | Cumulative Amount in Receptor, µg/cm² | | | | | |
| | Dc-05 | ±SD | Dc-07 | ±SD | Voltaren 1% | ±SD |
| 2 | 1.94 | 1.95 | 1.70 | 2.95 | 0.00 | 0.00 |
| 4 | 6.28 | 2.27 | 6.62 | 4.46 | 3.70 | 0.70 |
| 6 | 10.10 | 2.85 | 10.60 | 5.96 | 6.13 | 1.14 |

The above data show that skin permeation for diclofenac sodium and diclofenac diethylamine cream formulations was similar.

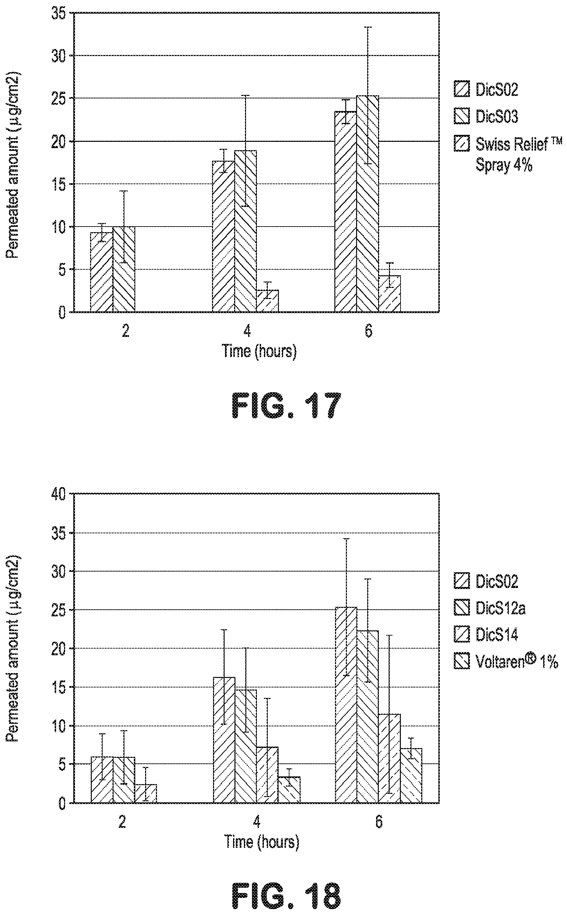

The experimental results obtained with a diclofenac spray composition using diclofenac sodium and diclofenac diethylamine are presented in Tables 33 and 34, below, and in FIGURE 17. The experimental procedure was the same as that for the previous spray compositions, except that the dermatomed cadaver skin was from the back of a human male, 79 years old.

**TABLE 33**

| Diclofenac Spray Composition | | |
|---|---|---|
| | Composition, wt.% | |
| Ingredients | DcS02 | DcS03 |
| Diclofenac Na | 1 | |
| Diclofenac diethylamine | | |
| Propylene glycol | 10 | 10 |
| Lauryl lactate | 3 | 3 |
| Lactic acid | 1.5 | 1.5 |
| Glyceryl monolaurate | 3 | 3 |
| Ethanol | 48.5 | 48.5 |
| Water | 33 | 33 |
| TOTAL | 100 | 100 |

**TABLE 34**

| Diclofenac Permeation Data | | | | | | |
|---|---|---|---|---|---|---|
| | Cumulative Amount in Receptor, µg/cm² | | | | | |
| Time, hours | DcS-02 | ±SD | DcS-03 | ±SD | Swiss Relief™ spray gel | ±SD |
| 2 | 9.29 | 1.06 | 9.98 | 4.16 | 0 | 0 |
| 4 | 17.69 | 1.30 | 18.91 | 6.47 | 2.57 | 0.93 |
| 6 | 23.43 | 1.38 | 25.33 | 7.99 | 4.31 | 1.44 |

The above data show that a diclofenac containing spray composition provided better skin permeation for diclofenac than a spray gel composition that has a relatively larger concentration of diclofenac.

The effects of propylene glycol, Brij 58 and lactic acid on diclofenac skin permeation were investigated. The experimental results obtained with a diclofenac spray composition using diclofenac sodium, Brij 58, and different levels of propylene glycol and lactic acid are presented in Tables 35 and 36, below, and in FIGURE 18. The experimental procedure was the same as that for the previous spray compositions, except that the dermatomed cadaver skin was from the thigh of a human male, 79 years old.

**TABLE 35**

| Diclofenac Spray Composition | | | | |
|---|---|---|---|---|
| | Composition, wt.% | | | |
| Ingredients | DcS02 | DcS12 | DcS12a | DcS14 |
| Diclofenac Na | | | 1 | |
| Propylene glycol | 10 | 15 | 10 | 10 |
| Lauryl lactate | 3 | 3 | 3 | 3 |
| Lactic acid | 1.5 | 1.5 | 1.5 | 0.5 |
| Brij 58¹ | | 3 | 3 | 3 |
| Glyceryl monolaurate | 3 | 3 | 3 | 3 |
| Ethanol | 48.5 | 25 | 43.5 | 34.5 |
| Water | 33 | 53.5 | 35 | 45 |
| TOTAL | 100 | 100 | 100 | 100 |

Composition DcS12 was cloudy.

**TABLE 36**

| Diclofenac Permeation Data | | | | | | | | |
|---|---|---|---|---|---|---|---|---|
| Time, hours | Cumulative Amount in Receptor, µg/cm² | | | | | | | |
| | DcS02 | ±SD | DcS12a | ±SD | DcS14 | ±SD | Voltaren 1% | ±SD |
| 2 | 5.98 | 2.94 | 5.97 | 3.42 | 2.47 | 2.17 | 0.00 | 0.00 |
| 4 | 16.33 | 6.10 | 14.68 | 5.39 | 7.24 | 6.34 | 3.36 | 1.11 |
| 6 | 25.37 | 8.82 | 22.33 | 6.67 | 11.52 | 10.23 | 7.10 | 1.33 |

Addition of a higher level of propylene glycol enhanced the water content but caused formulation DcS12 to precipitate. The above data show that a diclofenac spray composition with a lower level of lactic acid showed a lower level of skin permeation of diclofenac.

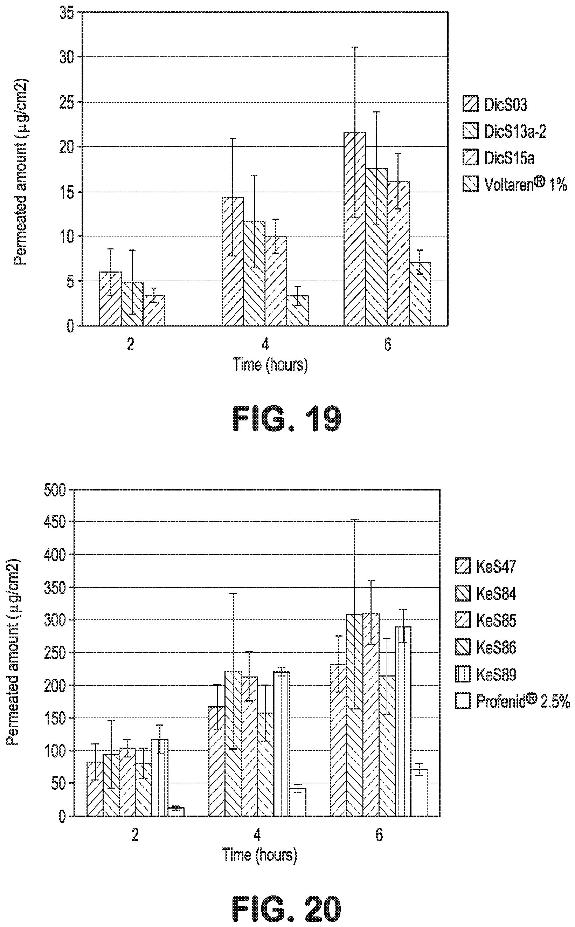

The effects of propylene glycol, Brij 58 and lactic acid on diclofenac skin permeation were investigated. The experimental results obtained with a diclofenac spray composition using diclofenac diethylamine, Brij 58, and different levels of propylene glycol and lactic acid are presented in Tables 37 and 38, below, and in FIGURE 19. The experimental procedure was the same as that for the previous spray compositions, except that the dermatomed cadaver skin was from the thigh of a human male, 79 years old.

**TABLE 37**

| Diclofenac Spray Composition | | | | | | |
|---|---|---|---|---|---|---|
| | Composition, wt.% | | | | | |
| Ingredients | DcS03 | DcS13 | DcS13a | DcS13a-2 | DcS15 | DcS15a |
| Diclofenac diethylamine | 1 | 1 | 1 | 1 | 1 | 1 |
| Propylene glycol | 10 | 15 | 10 | 15 | 10 | 10 |
| Lauryl lactate | 3 | 3 | 3 | 3 | 3 | 3 |
| Lactic acid | 1.5 | 1.5 | 1.5 | 1.5 | 0.5 | 0.5 |
| Brij 58¹ | 0 | 3 | 3 | 3 | 3 | 3 |
| Glyceryl monolaurate | 3 | 3 | 3 | 3 | 3 | 3 |
| Ethanol | 48.5 | 23 | 38 | 45 | 26 | 39.5 |
| Water | 33 | 55.5 | 40.5 | 28.5 | 53.5 | 40 |
| TOTAL | 100 | 105 | 100 | 100 | 100 | 100 |

| | | | | | | |
|---|---|---|---|---|---|---|
| Compositions DcS13, DcS13a and DCS15 were cloudy. | | | | | | |

**TABLE 38**

| Diclofenac Permeation Data | | | | | | | | |
|---|---|---|---|---|---|---|---|---|
| | Cumulative Amount in Receptor, µg/cm² | | | | | | | |
| Time, hours | DcS03 | ±SD | DcS13a-2 | ±SD | DcS15a | ±SD | Voltaren 1% | ±SD |
| 2 | 5.99 | 2.58 | 4.84 | 3.56 | 3.42 | 0.79 | 0.00 | 0.00 |
| 4 | 14.39 | 6.54 | 11.65 | 5.16 | 10.00 | 1.87 | 3.36 | 1.11 |
| 6 | 21.60 | 9.52 | 17.61 | 6.29 | 16.14 | 3.04 | 7.10 | 1.33 |

Formulations DcS13, DcS13a, and DcS15 were cloudy. The above data show that incorporation of Brij 58 reduced skin permeation of diclofenac.

The effects of propylene glycol and thickeners on ketoprofen skin permeation were investigated. The experimental results obtained with a ketoprofen spray formulation using ketoprofen, different levels of propylene glycol, and thickeners hydroxypropyl cellulose (100 cps) and hydroxypropyl cellulose (150-400 cps) are presented in Tables 39 and 40, below and in FIGURE 20. The experimental procedure was the same as that for the previous spray compositions, except that the dermatomed cadaver skin was from the thigh of a human male, 79 years old.

**TABLE 39**

| Ketoprofen and Thickeners Spray Composition | | | | | |
|---|---|---|---|---|---|
| | Composition, wt.% | | | | |
| Ingredients | KeS47 | KeS84 | KeS85 | KeS86 | KeS89 |
| Ketoprofen | 5 | 5 | 5 | 5 | 5 |
| Propylene glycol | 10 | 20 | 20 | 10 | 10 |
| Lauryl lactate | 3 | 3 | 3 | 3 | 3 |
| Lactic acid | 1.5 | 1.5 | 1.5 | 1.5 | 1.5 |
| HPC HY117¹² | 0 | 0 | 0.5 | 0.5 | 0 |
| HPC HY119¹³ | 0 | 0 | 0 | 0 | 0.25 |
| Glyceryl monolaurate | 3 | 3 | 3 | 3 | 3 |
| Ethanol | 37.5 | 27.5 | 27.5 | 32.5 | 32.25 |
| Water | 40 | 40 | 39.5 | 44.5 | 45 |
| TOTAL | 100 | 100 | 100 | 100 | 100 |

| | | | | | |
|---|---|---|---|---|---|
| ¹² hydroxypropyl cellulose (100 cps) ¹³ hydroxypropyl cellulose (150-400 cps) | | | | | |

**TABLE 40**

| Permeation Data | | | | | | | | | | | | |
|---|---|---|---|---|---|---|---|---|---|---|---|---|
| | Cumulative Amount in Receptor, µg/cm² | | | | | | | | | | | |
| Time, hours | KeS47 | ±SD | KeS84 | ±SD | KeS85 | ±SD | KeS86 | ±SD | KeS89 | ±SD | Profenid 2.5% | ±SD |
| 2 | 82.63 | 27.60 | 94.36 | 51.62 | 104.05 | 13.26 | 80.82 | 22.75 | 117.71 | 21.55 | 12.75 | 3.00 |
| 4 | 167.45 | 34.34 | 221.64 | 118.40 | 213.69 | 37.65 | 157.90 | 42.69 | 221.11 | 6.86 | 42.43 | 5.90 |
| 6 | 232.80 | 42.90 | 309.28 | 144.22 | 311.38 | 48.99 | 214.48 | 57.66 | 290.61 | 25.07 | 71.36 | 8.79 |

All formulations above gave clear solutions. The above data show that KeS84, KeS85, and KeS89 all exhibited significant permeation enhancement compared to KeS47. KeS86, with lower propylene glycol, showed similar permeation to KeS47.

## Claims

1. A sprayable solution which comprises a nonsteroidal anti-inflammatory drug (NSAID); lauryl lactate; lactic acid; glyceryl monolaurate; ethanol; and water, wherein the NSAID is
a) a propionic acid derivative selected from the group consisting of ketoprofen, ibuprofen, naproxen, and pharmaceutically acceptable salts thereof; or
b) an acetic acid derivative selected from the group consisting of diclofenac, indomethacin, etodolac, and pharmaceutically acceptable salts thereof.

2. The sprayable solution in accordance with claim 1 further comprising a non-ionic surfactant having an HLB value of at least 12.

3. The sprayable solution in accordance with claim 2 wherein the non-ionic surfactant is an alkoxylated alcohol.

4. The sprayable solution in accordance with claim 1 wherein the NSAID is ketoprofen.

5. The sprayable solution in accordance with claim 1 wherein the water:ethanol weight ratio is in the range of about 0.3:1 to about 2.6:1.

6. The sprayable solution in accordance with claim 1 wherein the lauryl lactate is present in an amount in the range of about 1 to about 5 weight percent, based on the total weight of the solution.

7. The sprayable solution in accordance with claim 1 being a sprayable clear solution which comprises, based on total weight of the solution, the nonsteroidal anti-inflammatory drug (NSAID) in an amount in the range of about 1 to about 10 weight percent,
lauryl lactate in an amount in the range of about 1 to about 5 weight percent,
lactic acid in an amount in the range of about 0.5 to about 5 weight percent; glyceryl monolaurate in an amount in the range of about 2 to about 5 weight percent;
propylene glycol in an amount in the range of about 5 to about 30 weight percent;
alkoxylated alcohol having a HLB value of at least 12 in an amount in the range of 0 to about 7 percent; and
the remainder a mixture of water and ethanol in a respective weight ratio in the range of about 0.3:1 to about 2.6:1.

8. The sprayable clear solution in accordance with claim 7 which comprises about 5 weight percent ketoprofen;
about 3 weight percent lauryl lactate;
about 1.5 weight percent lactic acid;
about 3 weight percent glyceryl monolaurate;
about 3 weight percent polyethylene glycol ether of cetyl alcohol having a HLB value of about 15.7 and represented by the formula CH₃(CH₂)₁₄CH₂(OCH₂CH₂)ₙOH where n has an average value 20;
about 10 weight percent propylene glycol; and the remainder a water-ethanol mixture in a respective weight ratio of about 1.7.

## Patentansprüche

1. Sprühbare Lösung, die ein nicht-steroidales antiinflammatorisches Arzneimittel (NSAID), Lauryllactat, Milchsäure, Glycerylmonolaurat, Ethanol und Wasser umfasst,
wobei das NSAID
a) ein Propionsäure-Derivat, ausgewählt aus der Gruppe, bestehend aus Ketoprofen, Ibuprofen, Naproxen und pharmazeutisch verträglichen Salzen davon, oder
b) ein Essigsäure-Derivat, ausgewählt aus der Gruppe, bestehend aus Diclofenac, Indomethacin, Etodolac und pharmazeutisch verträglichen Salzen davon, ist.

2. Sprühbare Lösung gemäß Anspruch 1, die ferner ein nicht-ionisches Tensid mit einem HLB-Wert von mindestens 12 umfasst.

3. Sprühbare Lösung gemäß Anspruch 2, wobei das nicht-ionische Tensid ein alkoxylierter Alkohol ist.

4. Sprühbare Lösung gemäß Anspruch 1, wobei das NSAID Ketoprofen ist.

5. Sprühbare Lösung gemäß Anspruch 1, wobei das Wasser:Ethanol-Gewichtsverhältnis in dem Bereich von etwa 0,3:1 bis etwa 2,6:1 liegt.

6. Sprühbare Lösung gemäß Anspruch 1, wobei das Lauryllactat in einer Menge in dem Bereich von etwa 1 bis etwa 5 Gew.-%, basierend auf dem Gesamtgewicht der Lösung, vorhanden ist.

7. Sprühbare Lösung gemäß Anspruch 1, die eine sprühbare, klare Lösung ist, umfassend, basierend auf dem Gesamtgewicht der Lösung, das nicht-steroidale antiinflammatorische Arzneimittel (NSAID) in einer Menge in dem Bereich von etwa 1 bis etwa 10 Gew-%,
Lauryllactat in einer Menge in dem Bereich von etwa 1 bis etwa 5 Gew.-%,
Milchsäure in einer Menge in dem Bereich von etwa 0,5 bis etwa 5 Gew.-%,
Glycerylmonolaurat in einer Menge in dem Bereich von etwa 2 bis etwa 5 Gew.-%,
Propylenglycol in einer Menge in dem Bereich von etwa 5 bis etwa 30 Gew.-%,
alkoxylierten Alkohol mit einem HLB-Wert von mindestens 12 in einer Menge in dem Bereich von 0 bis etwa 7%, und
den Rest, der ein Gemisch von Wasser und Ethanol in einem entsprechenden Gewichtsverhältnis in dem Bereich von etwa 0,3:1 bis etwa 2,6:1 ist.

8. Sprühbare, klare Lösung gemäß Anspruch 7, die etwa 5 Gew.-% Ketoprofen,
etwa 3 Gew.-% Lauryllactat,
etwa 1,5 Gew.-% Milchsäure,
etwa 3 Gew.-% Glycerylmonolaurat,
etwa 3 Gew.-% Polyethylenglycolether von Cetylalkohol, der einen HLB-Wert von etwa 15,7 aufweist und durch die Formel CH₃(CH₂)₁₄CH₂(OCH₂CH₂)ₙOH repräsentiert wird,
worin n einen durchschnittlichen Wert von 20 aufweist,
etwa 10 Gew.-% Propylenglycol und den Rest umfasst, der ein Wasser-Ethanol-Gemisch in einem entsprechenden Gewichtsverhältnis von etwa 1,7 ist.

## Revendications

1. Solution pulvérisable qui comprend un anti-inflammatoire non stéroïdien (AINS); du lactate de lauryle; de l'acide lactique ; du monolaurate de glycéryle ; de l'éthanol ; et de l'eau, dans laquelle l'AINS est
a) un dérivé de l'acide propionique choisi dans le groupe constitué du kétoprofène, de l'ibuprofène, du naproxène, et des sels pharmaceutiquement acceptables de ceux-ci ; ou
b) un dérivé de l'acide acétique choisi dans le groupe constitué du diclofénac, de l'indométhacine, de l'étodolac, et des sels pharmaceutiquement acceptables de ceux-ci.

2. Solution pulvérisable selon la revendication 1 comprenant en outre un tensioactif non ionique ayant une valeur HLB d'au moins 12.

3. Solution pulvérisable selon la revendication 2 dans laquelle le tensioactif non ionique est un alcool alcoxylé.

4. Solution pulvérisable selon la revendication 1 dans laquelle l'AINS est du kétoprofène.

5. Solution pulvérisable selon la revendication 1 dans laquelle le rapport pondéral eau/éthanol est dans la plage d'environ 0,3/1 à environ 2,6/1.

6. Solution pulvérisable selon la revendication 1 dans laquelle le lactate de lauryleest présent en une quantité dans la plage d'environ 1 à environ 5 pour cent en poids, sur la base du poids total de la solution.

7. Solution pulvérisable selon la revendication 1 étant une solution transparente pulvérisable qui comprend, sur la base du poids total de la solution, l'anti-inflammatoire non stéroïdien (AINS) en une quantité dans la plage d'environ 1 à environ 10 pour cent en poids,
du lactate de lauryleen une quantité dans la plage d'environ 1 à environ 5 pour cent en poids,
de l'acide lactique en une quantité dans la plage d'environ 0,5 à environ 5 pour cent en poids ;
du monolaurate de glycéryle en une quantité dans la plage d'environ 2 à environ 5 pour cent en poids ;
du propylène glycol en une quantité dans la plage d'environ 5 à environ 30 pour cent en poids ;
de l'alcool alcoxylé ayant une valeur HLB d'au moins 12 en une quantité dans la plage de 0 à environ 7 pour cent; et
le reste étant un mélange d'eau et d'éthanol en un rapport pondéral respectif dans la plage d'environ 0,3/1 à environ 2,6/1.

8. Solution transparente pulvérisable selon la revendication 7 qui comprend environ 5 pour cent en poids de kétoprofène ;
environ 3 pour cent en poids de lactate de lauryle;
environ 1,5 pour cent en poids d'acide lactique ;
environ 3 pour cent en poids de monolaurate de glycéryle ;
environ 3 pour cent en poids d'éther de polyéthylène glycol d'alcool cétylique ayant une valeur HLB d'environ 15,7 et représenté par la formule CH₃(CH₂)₁₄CH₂(OCH₂CH₂)ₙOH où n a une valeur moyenne de 20 ;
environ 10 pour cent en poids de propylène glycol ; et le reste étant un mélange eau-éthanol en un rapport pondéral respectif d'environ 1,7.
